(19) Europäisches Patentamt
European Patent Office
Office européen des brevets

(11) Numéro de publication: **0 206 957 A1**

(12) **DEMANDE DE BREVET EUROPEEN**

(21) Numéro de dépôt: **86420158.7**

(51) Int. Cl.⁴: **C07C 45/49** , C07C 47/52

(22) Date de dépôt: **20.06.86**

(30) Priorité: **21.06.85 FR 8509676**

(43) Date de publication de la demande: **30.12.86 Bulletin 86/52**

(84) Etats contractants désignés: **AT BE CH DE FR GB IT LI LU NL SE**

(71) Demandeur: **RHONE-POULENC SPECIALITES CHIMIQUES**
**"Les Miroirs" 18, Avenue d'Alsace**
**F-92400 Courbevoie(FR)**

(72) Inventeur: **Basset, Jean-Marie**
**21, petite rue de la Doua**
**F-69100 Villeurbanne(FR)**
Inventeur: **Mutin, Robert**
**21, rue E. Renan**
**F-69330 Meyzieu(FR)**

(74) Mandataire: **Cazes, Jean-Marie et al**
**RHONE-POULENC INTERSERVICES Service Brevets Chimie Centre de Recherches de Saint-Fons B.P. 62**
**F-69192 Saint-Fons Cédex(FR)**

# (54) Procédé de préparation d'aldéhydes aromatiques.

(57) Procédé de préparation d'aldéhydes aromatiques par hydrocarbonylation de composés chloro-ou bromoaromatiques en présence d'un accepteur d'hydracide, d'un complexe palladium/phosphine et éventuellement d'une phosphine caractérisé en ce que l'on fait réagir un mélange d'oxyde de carbone et d'hydrogène avec des chloro-ou bromoarènechrometricarbonyles.

EP 0 206 957 A1

# PROCEDE DE PREPARATION D'ALDEHYDES AROMATIQUES

La présente invention a pour objet un procédé de préparation d'aldéhydes aromatiques par hydrocarbonylation de composés aromatiques halogénés en présence d'un complexe d'un métal noble.

En raison de l'abondance et du faible coût de l'oxyde de carbone l'industrie s'intéresse à la production d'aldéhyde aromatique par hydrocarbonylation des halogénures aromatiques. A cet effet A. SCHOENBERG et R. F. HECK, J. Am. Chem. Soc. 96 7761-7764 (1974) ont proposé de faire réagir un mélange d'oxyde de carbone et d'hydrogène avec des iodures et bromures aromatiques en présence d'une amine jouant le rôle d'accepteur d'hydracide et d'un dihalogénodiphosphinopalladium comme catalyseur, (cf. également brevet américain No. 3.960.932). Dans la demande de brevet allemand No. 3.242.582 on a proposé un perfectionnement à ce procédé consistant à opérer en présence d'une phosphine ou d'un phosphite libre pour augmenter la productivité de la réaction. En dépit de leur intérêt ces procédés souffrent de l'inconvénient majeur, au plan industriel, d'impliquer la mise en oeuvre de bromures ou d'iodures d'aryle pour obtenir de bons rendements en aldéhydes aromatiques ; il s'est avéré en effet que les chlorures d'aryles conduisent à de faibles rendements en aldéhydes en raison de la faible réactivité de la liaison chlore-carbone du cycle aromatique même dans des conditions dures de pression et de température (cf. H. YOSHIBA et al, Bull. Chem. Soc. Jap. 49[1976]). Le problème de l'hydrocarbonylation des chlorures d'aryles moins coûteux que les bromures et les iodures reste par conséquent à résoudre. La présente invention a précisément pour objet la solution du problème posé par l'activation de la liaison chlore/carabone d'un cycle aromatique de façon à rendre possible la carbonylation des chlorures aromatiques. Par ailleurs la présence de substituants électrodonneurs sur le reste aromatique des bromures aromatiques contribue à diminuer quelque peu la réactivité de la liaison brome/carbone du cycle aromatique, ce qui se traduit par des rendements en aldéhydes inférieurs. Un autre objet de la présente invention réside par conséquent dans un procédé d'hydrocarbonylation de bromures aromatiques comportant des substituants électrodonneurs.

Plus spécifiquement la présente invention a pour objet un procédé de préparation d'aldéhydes aromatiques par hydrocarbonylation de composés chloro-ou bromoaromatiques en présence d'un accepteur d'hydracide, d'un complexe palladium/phosphine et éventuellement d'une phosphine caractérisé en ce que l'on fait réagir un mélange d'oxyde de carbone et d'hydrogène avec des chloro-ou bromoarènechromecarbonyles.

On a constaté de manière inattendue que la présence d'un reste chromecarbonyle $-Cr(CO)_3$ sur le noyau aromatique des chlorures aromatiques et des bromures aromatiques à substituants électrodonneurs active notablement la liaison halogène-carbone et permet d'améliorer les rendements en aldéhydes aromatiques.

Les composés arènechrometricarbonyles sont des produits connus qui peuvent être obtenus aisément par diverses voies de synthèse (cf. G. WILKINSON et al, Comprehensive Organometallic Chemistry, vol. 3 pages 1001-1021, édité par Pergamon Press). Une voie d'accès préférée aux arènechrometricarbonyles consiste à faire réagir un composé aromatique avec le chromehexacarbonyle dans divers solvants organiques tels que des hydrocarbures et/ou des éthers ou au sein d'un excès du composé aromatique de départ. Cette méthode convient tout particulièrement bien à la synthèse d'arènechrometricarbonyles comportant des substituants électrodonneurs.

Plus spécifiquement encore la présente invention a pour objet un procédé de préparation d'aldéhydes aromatiques répondant à la formule générale:

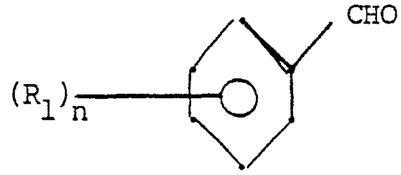

(I)

par hydrocarbonylation de composés halogénoarènechrometricarbonyles de formule générale:

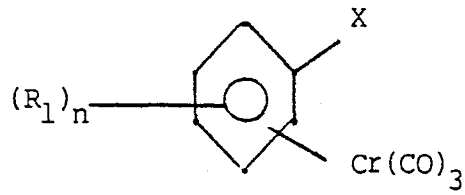

(II)

dans lesquelles:

-$R_1$ représente un radical alkyle ayant de 1 à 20 atomes de carbone ; un radical alkoxy de 1 à 20 atomes de carbone ; un atome de fluor ; un reste alkyle mono-ou polyhalogéné ; un groupe formyle ; un groupe carbonyloxyalkyle ; un groupe amino tertiaire, un groupe hydroxyle ; un reste acyle ; un reste acylamine,

-n représente un nombre entier de 1 à 3 et de préférence de 1 à 2;

-X représente un atome de chlore ou de brome.

Comme exemples spécifiques de radicaux

$R_1$ on peut citer à titre non limitatif des radicaux méthyle, éthyle, n-pro pyle, isopropyle, n-butyle, iosbutyle, sec-butyle, n-pentyle, éthyl-2 hexyl e, dodécyle, octadécyle, cyclohexyle, méthylcyclohexyle, benzyle, phényl-2 éthyle, des radicaux alkoxy tels que méthoxy, éthoxy, n-propoxy, isopropox y, n-butyloxy, éthyl-2 hexyloxy, dodécyloxy, hexadécyloxy ; des radicaux ha logéno alkyles tels que trifluorométhyle, difluorochlorométhyle, perfluoroé thyle ; des groupes carbonyloxyalkyle dans lesquels le reste alkyle comport e de 1 à 4 atomes de carbone tels que les groupes carbonyloxyméthyle, carbo nyloxyéthyle, carbonyloxyisopropyle ; des restes amino tertiaires de formul e

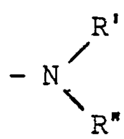

dans laquelle R' et R" identiques ou différents représentent des radicaux alkyles ayant de 1 à 20 atomes de carbones tels que ceux cités pour $R_1$ des radicaux arylalkyles, des radicaux phényles ; des restes acyle et acylamino de formules

R"'-CO - et 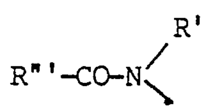

dans lesquelles R"' représente des radicaux alkyles, cycloalkyles ou arylalkyles tels que ceux cités par R et des radicaux aryles (phényle, toluyle, naphtyles). Les radicaux acétyle, propionyle, benzoyle, méthylbenzoyles, acétylméthylamino, éthylbenzoylamino représentent des exemples spécifiques de radicaux acyles et acylamino. De préférence lorsque X représente un atome de brome $R_1$ représente un reste électrodonneur.

De préférence $R_1$ représente un radical alkyle inférieur, c'est-à-dire un radical alkyle ayant de 1 à 4 atomes de carbone.

Comme exemples d'halogénoarènechrometricarbonyles on peut citer plus particulièrement :

le chlorobenzènechrometricarbonyle,

le chlorotoluènechrometricarbonyle,

le bromotoluènechrometricarbonyle,

le fluorochlorobenzènechrometricarbonyle,

le méthyl-1 fluoro-4 chloro-2 benzènechrometricarbonyle,

le chloro-1 trifluorométhyl-4 benzènechrometricarbonyle,

le chloro-1 tertiobutyl-4 benzènechrometricarbonyle,

le chloro-1 méthoxy-4 benzènechrometricarbonyle,

le bromo-1 méthoxy-4 benzènechrometricarbonyle,

le chloro-1 méthoxy-2 benzènechrometricarbonyle,

le chloro-1 hydroxy-4 benzènechrometricarbonyle,

le chloro-1 hydroxy-2 benzènechrometricarbonyle,

le bromo-1 diéthylamino-4 benzènechrometricarbonyle,

le chloro-1 méthoxycarbonyl-3 benènechrometricarbonyle,

le bromo-2 méthoxycarbonyl-3 benzènechrometricarbonyle,

le chloro-1 acétyl-4 benzènechrometricarbonyle.

Le procédé selon l'invention convient tout spécialement bien à la préparation d'aldéhydes tels que le benzaldéhyde, le p-hydroxybenzaldéhyde, le p-méthoxybenzaldéhyde, le p-tolualdéhyde, le p-trifluorométhylbenzaldéhyde, le p-trifluorométhoxybenzaldéhyde.

Pour la mise en oeuvre du procédé selon l'invention on peut faire appel à tout accepteur d'hydracide soluble dans le milieu ou miscible avec les composants du milieu réactionnel et ne libérant pas d'eau lors de la réaction avec l'hydracide formé. On peut utiliser des oxydes alcalino-terreux (CaO) ; des carbonates et bicarbonates alcalins ou alcalino-terreux (carbonate et bicarbonate de sodium) ; des carboxylates alcalins ou alcalino-terreux dérivés d'acides carboxyliques faibles (acides carboxyliques ayant un pK dans l'eau à 25°C supérieur à 4) tels que les acétates, propionates, butyrates, isobutyrates, benzoates, phénylacétates de sodium ou de potassium ; des amines tertiaires et des bases hétérocycliques azotées tertiaires telles que la triméthylamine, la triéthylamine, la méthyltriéthylamine, la pyridine, les picolines, les N-alkylpipéridines (N-méthyl-ou N-éthylpipéridines par exemple). Les amines tertiaires et les bases azotées hétérocycliques tertiaires constituent une classe préférée d'accepteurs d'hydracides.

La quantité d'accepteur d'hydracide est au moins voisine de la quantité stoechiométrique nécessaire à la neutralisation de l'hydracide formé au cours de la réaction, c'est-à-dire voisine d'au moins 1 mole par mole d'halogénoarènechrometricarbonyle. De préférence on met en oeuvre au moins 1,1 mole d'accepteur d'hydracide par mole d'halogénoarènechrometricarbonyle. Il n'y a pas de limite supérieure critique de la quantité d'accepteur d'hydracide et dans le cas des bases azotées tertiaires ces dernières peuvent constituer le milieu réactionnel. Pour les autres accepteurs d'hydracides il n'est en général pas nécessaire de recourir à plus de 1,5 mole d'accepteur par mole d'halogénoarènechrometricarbonyle ; une quantité comprise dans un intervalle de 1 à 1,25 est en général satisfaisante.

Les complexes palladium/phosphines utilisés comme catalyseurs dans le procédé selon la présente invention sont ceux habituellement mis en oeuvre dans les réactions d'hydrocarbonylation des halogénures aromatiques (cf. A. SCHOENBERG et al, loc. cit., H. YOSHIDA et al, loc. cit. ; brevet américain No. 3.960.932 et demande allemande No. 3.242.582). On peut donc faire appel indifféremment à des complexes du palladium zéro ou à des complexes du palladium divalent ; ces derniers sont en général préférés en raison de leur stabilité à l'air qui les rend plus faciles à manipuler. Plus pécifiquement on peut faire appel à des complexes du palladium de formules générales :

$Pd(CO)P[(R_2)_3]_3$ (IV)

$Pd_3(CO)_3[P(R_2)_3]_m$ (V)

$Pd[P(R_2)_3]_4$ (VI)

$PdZ_2[P(R_2)_3]_2$ (VII)

dans lesquelles :

-$R_2$ représente un radical hydrocarboné aliphatique ou cycloaliphatique saturé ou aromatique comportant de 1 à 20 atomes de carbone ;

-Z représente un atome d'halogène ou un reste acyloxy de formule $R_3$-COO-dans laquelle $R_3$ représente un radical alkyle, cycloalkyle, arylalkyle ayant de 1 à 20 atomes de carbone tel que ceux cités plus haut pour R ou aryle ;

-m est 3 ou 4.

Dans les formules (IV) à (VII) les radicaux $R_2$, qui peuvent être identiques ou différents, représentent plus spécialement des radicaux méthyle, éthyle, propyle, isopropyle, n-butyle, sec-butyle, n-octyle, cyclohexyle, méthylcyclohexyle, phényle, toluyles, éthylphényles, xylyles, naphtyles, benzyle, phényl-2 éthyle. De préférence un au moins des radicaux $R_2$ représente un radical électrodonneur et plus préférentiellement encore un radical aryle. Z représente de préférence un atome de chlore ou de brome ou un radical acyloxyaliphatique saturé inférieur (acétoxy, propionyloxy, butyryloxy) ou un reste acyloxyaromatique - (benzoyloxy, toluyloxy). Comme exemple de complexes palladium/phosphine on peut citer à titre non limitatif le tris-(méthyldiphénylphosphino)-palladiummonocarbonyle, le

tris-(triphénylphosphino)palladiummonocarbonyle, le

tris-(éthylditolylphosphino)palladiummonocarbonyle, le

tris-(éthylditolylphosphino)palladium, le

tétrakis-(tritolylphosphino)palladium, le

dichloro-bis-(triphénylphosphino)palladium, le

dichloro-bis-(tritolylphosphino)palladium, le

diisopropionyloxy-bis-(triphénylphosphino)-palladium. On fait appel de préférence aux dihalogéno-bis-(triarylphosphino)palladium et aux diacyloxy-bis-(triarylphosphino)palladium.

Le procédé selon l'invention est conduit en présence d'un solvant des divers composés participant à la réaction, inerte dans les conditions de la réaction et pratiquement anhydre. Comme cela a été indiqué plus haut un premier groupe de solvants utilisables à cet effet est constitué par les bases tertiaires azotées utilisées comme accepteur d'hydracide. On peut également faire appel à d'autres composés organiques tels que les hydrocarbures aliphatiques saturés (hexane par exemple), cycloaliphatiques saturés (cyclohexane, méthylcyclohexane), aromatiques (benzène, toluène, xylènes, éthylbenzène), des hydrocarbures aromatiuqes halogénés (chlorobenzène, chlorotoluènes), des éthers aliphatiques, des éthers cycliques (tétrahydrofuranne), aromatiques (anisole, chloroanisoles, bromoanisoles), des lactames (N-méthylpyrrolidone), des esters d'acides mono-ou dicarboxyliques (adipate de méthyle, téréphtalate de méthyle), des éthers de polyols (éther diméthylique de l'éthylèneglycol ou du diéthylèneglycol). D'une manière générale on fait appel à des composés organiques dont le caractère électrodonneur est le plus faible possible. D'un point de vue pratique il s'avère particulièrement avantageux d'utiliser comme solvant ou co-solvant le dérivé halogénoaromatique dont dérive l'halogénoarènechrometricarbonyle lorsqu'il est liquide dans les conditions normales de pression et de température. On prépare alors ce dernier par réaction du chromehexacarbonyle avec un excès de composé halogénoaromatique ; la solution d'halogénoarènechrometricarbonyle ainsi obtenue est directement utilisée dans la réaction d'hydrocarbonylation.

La concentration des réactifs dans le milieu réactionnel n'est pas critique et peut varier dans de larges limites.

La quantité de catalyseur au palladium exprimée en nombre d'atome-gramme de palladium par mole d'halogénoarènechrometricarbonyle peut varier dans de larges limites. En général elle peut être comprise dans un intervalle allant de 1 x $10^{-5}$ à 0,5 atome-gramme de palladium par mole d'halogénoarènechrometricarbonyles ; des quantités allant de 1 x $10^{-4}$ à 1 x $10^{-1}$ at.g de palladium par mole d'halogénoarènechromatricarbonyles conviennent bien.

L'hydrocarbonylation des halogénoarènechrometricarbonyles est de préférence conduite en présence d'une phosphine libre qui est avantageusement la même que celle liée au palladium du catalyseur. D'une manière générale, on peut utiliser les phosphines citées dans la demande allemande No. 3.242.582. La quantité de phosphine exprimée en at.g de phosphore par at.g de palladium peut varier dans de larges limites en fonction de la concentration en palladium dans le milieu ; le rapport phosphine libre/palladium est de préférence d'autant plus élevé que la concentration en palladium dans le milieu est plus faible. On a recours de façon générale à une quantité de phosphine telle que le nombre d'at.g de phosphore par at.g de palladium soit compris dans un intervalle de 0.5 à 1000 et de préférence de 2 à 100.

On pourrait, sans sortir du cadre de la présente invention, générer "in situ" les espèces catalytiques nécessaires au déroulement de la réaction en chargeant dans le milieu réactionnel un complexe du palladium(+2) ou un sel d'acides carboxyliques du palladium(+2) (acétate palladeux, propionate palladeux) et la quantité adéquate de phosphine libre.

La réaction peut être conduite dans un large intervalle de températures. En général une température comprise dans un intervalle de 50 à 250°C convient bien. De préférence on opère à une température de 80 à 200°C.

Le procédé selon l'invention peut être mis en oeuvre sous une pression d'oxyde de carbone égale à la pression atomosphérique ou sous pression plus élevée. De préférence la pression d'oxyde de carbone est inférieure à 150 bar et plus préférentiellement encore à 80 bar. Des pressions comprises entre 1 et 50 bar conviennent bien.

Le rapport molaire oxyde de carbone/hydrogène n'est pas critique et peut varier dans de larges limites. Ainsi on peut faire appel à un large excès de l'un ou l'autre de ces réactifs. Plus particulièrement le rapport $CO/H_2$ peut varier dans un intervalle de 0,1 à 10.

D'un point de vue pratique le procédé est réalisé simplement par passage d'un courant d'oxyde de carbone et d'hydrogène dans une solution organique de l'halogénoarènechrometricarbonyle et des autres composés nécessaires au déroulement de la réaction ou par action d'une pression d'oxyde de carbone et d'hydrogène sur ladite solution, dans un récipient agité résistant à la pression. En fin de réaction les divers constituants du milieu réactionnel sont séparés par les techniques usuelles. Selon les conditions de la réaction une partie plus ou moins importante de l'aldéhyde aromatique formé est présente sous forme d'arènechrometricarbonyle. Il suffit alors de porter le mélange réactionnel à température élevée et sous une pression d'oxyde de carbone suffisante pour provoquer la décoordination du chrome et de l'aldéhyde aromatique que l'on peut récupérer.

Les exemples suivants illustrent l'invention et montrent comment elle peut être mise en pratique. Les halogénoarènechrometricarbonyles utilisés dans les exemples ont été préparés par chauffage à reflux d'un composé halogénoaromatique avec le chromehexacarbonyle dans un mélange de tétrahydrofuranne et d'oxyde de butyle.

EXEMPLE 1

Dans un autoclave en acier inoxydable de 100 ml équipé d'un système de chauffage et d'un agitateur magnétique, on charge :

-1 m.mole de chlorobenzènechrometricarbonyle

-0,02 m.mole de $PdCl_2[P(C_6H_5)_3]_2$

-0,1 m.mole de triphénylphosphine

-20 ml de toluène

-0,16 ml (1,2 m.mole) de triéthylamine.

L'autoclave est purgé par un courant de CO puis fermé. On charge ensuite de l'oxyde de carbone jusqu'à une pression de 15 bar puis 15 bar d'hydrogène, puis porte le mélange des réactifs à 120°C sous agitation et maintient ces conditions pendant 5 heures. Après quoi le contenu de l'autoclave est refroidi à 20°C puis dégazé.

On prélève une partie aliquote de masse réactionnelle sur laquelle on identifie les produits formés par spectrographie de masse et RMN du proton. Le benzaldéhyde est dosé par chromatographie en phase gazeuse à ionisation de flamme. On constate qu'il s'est formé 0,54 m.mole de benzaldéhyde, ce qui représente 54 % du chlorobenzènetricarbonyle chargé initialement - (rendement réel).

On dose encore dans le milieu réactionnel 0,15 m.mole de formylarènechrometricarbonyle ce qui représente 15 % du chlorobenzènetricarbonyle chargé. Au total le benzaldéhyde formé représente 69 % du chlorobenzènechrometricarbonyle chargé.

EXEMPLE 2

Dans l'appareil de l'exemple 1, on a chargé 0,7 m.mole de chlorobenzènechrometricarbonyle, 0,014 m.mole de dichloro-bis(triphénylphosphino)-palladium, 0,84 m.mole de triéthylamine, 0,07 m.mole de triphénylphosphine et 22,5 g de chlorobenzène. L'autoclave est purgé par un courant d'oxyde de carbone, fermé puis chargé avec 1 bar d'oxyde de carbone puis 1 bar d'hydrogène. La température est portée à 130°C sous agitation. On maintient 24 h dans ces conditions puis refroidit la masse réactionnelle à 20°C et dégaze l'autoclave.

Le benzaldéhyde formé est dosé comme à l'exemple 1. Le rendement réel s'élève à 65 %.

EXEMPLE 3

On a opéré comme à l'exemple 2 mais sous une pression de 15 bar de CO et de 15 bar d'hydrogène.

On a obtenu un rendement en benzaldéhyde de 52 %.

EXEMPLES 4 à 7

On opère dans les conditions de l'exemple 3 en remplaçant le chlorobenzènechrometricarbonyle par divers chloroarènechromecarbonyles. On a obtenu les résultats consignés dans le tableau suivant :

| EXEMPLES | SUBSTRAT | ALDEHYDE | RR (1) % |
|---|---|---|---|
| 4 | chloro-1 méthyl-4 benzène-chrometricarbonyle | p-tolualdéhyde | 36 |
| 5 | chloro-1 trifluorométhyl-4 benzènechrometricarbonyle | trifluorométhyl-4 benzaldéhyde | 20 |
| 6 | chloro-1 méthoxy-4 benzènechrometricarbonyle | p-méthoxybenzaldéhyde | 30 |
| 7 | bromo-1 diméthylamino-4 benzènechrometricarbonyle | p-diméthylamino-benzaldéhyde | 35 |

## Revendications

1°) Procédé de préparation d'aldéhydes aromatiques par hydrocarbonylation de composés chloro-ou bromoaromatiques en présence d'un accepteur d'hydracide, d'un complexe palladium/phosphine et éventuellement d'une phosphine caractérisé en ce que l'on fait réagir un mélange d'oxyde de carbone et d'hydrogène avec des chloro-ou bromoarènechrometricarbonyles.

2°) Procédé selon la revendication 1, caractérisé en ce que l'on prépare des aldéhydes de formule générale :

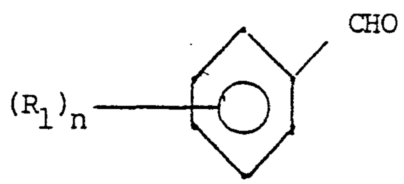

(I)

par carbonylation de composés halogénoarènechrometricarbonyles de formule générale :

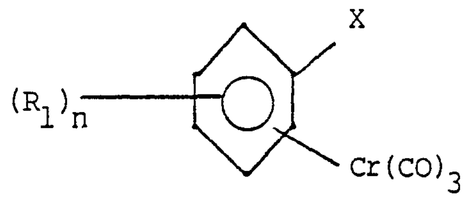

(II)

dans lesquelles :

-$R_1$ représente un radical alkyle ayant de 1 à 20 atomes de carbone ; un radical alkoxy de 1 à 20 atomes de carbone ; un atome de fluor ; un reste alkyle mono-ou polyhalogéné ; un groupe formyle ; un groupe carbonyloxyalkyle ; un groupe amino tertiaire, un groupe hydroxyle ; un reste acyle ; un reste acylamine,

-n représente un nombre entier de 1 à 3 et de préférence de 1 à 2;

-X représente un atome de chlore ou de brome.

3°) Procédé selon la revendication 3, caractérisé en ce que dans les formules (I) et (II) $R_1$ représente un radical alkyle inférieur.

4°) Procédé selon l'une quelconque des revendications 1 à 3, caractérisé en ce que l'accepteur d'hydracide est pris dans le groupe formé par les oxydes et hydroxydes alcalino-terreux, les carbonates ou bicarbonates alcalins ou alcalino-terreux, les carboxylates alcalins ou alcalino-terreux, les amines tertiaires, les bases hétérocycliques azotées tertiaires.

5°) Procédé selon l'une quelconque des revendications 1 à 4, caractérisé en ce que la quantité d'accepteur d'hydracide est d'au moins environ 1 mole par mole d'halogénoarènechrometricarbonyle.

6°) Procédé selon l'une quelconque des revendications 1 à 5, caractérisé en ce que l'on utilise comme catalyseur des complexes palladium/phosphine pris dans le groupe formé par les composés de formules :

$Pd(CO)P[(R_2)_3]_3$ (IV)

$Pd_3(CO)_3[P(R_2)_3]_m$ (V)

$Pd[P(R_2)_3]_4$ (VI)

$PdZ_2[P(R_2)_3]_2$ (VII)

dans lesquelles :

-$R_2$ représente un radical hydrocarboné aliphatique ou cycloaliphatique saturé ou aromatique comportant de 1 à 20 atomes de carbone ;

-Z représente un atome d'halogène ou un reste acyloxy de formule $R_3$-COO-dans laquelle $R_3$ représente un radical alkyle, cycloalkyle, arylalkyle ayant de 1 à 20 atomes de carbone tel que ceux cités plus haut pour R ou aryle ;

-m est 3 ou 4.

7°) Procédé selon la revendication 6, caractérisé en ce que l'on utilise comme catalyseurs des composés de formules (IV) à (VII) dans lesquelles l'un au moins des radicaux $R_2$ représente un radical aryle.

8°) Procédé selon la revendication 7, caractérisé en ce que l'on utilise le dichloro-bis-(triphénylphosphino)palladium comme catalyseur.

9°) Procédé selon l'une quelconque des revendications 1 à 8, caractérisé en ce que la quantité de catalyseur exprimée en atome-gramme de palladium par mole d'halogénoarènechrometricarbonyle est comprise dans un intervalle de 1 x $10^{-5}$ à 0,5 atome-gramme par mole d'halogénoarènechrometricarbonyle.

10°) Procédé selon l'une quelconque des revendications 1 à 9, caractérisé en ce que l'on opère en présence d'une quantité de phosphine libre exprimée en atome-gramme de phosphore par atome-gramme de palladium comprise dans un intervalle de 0,5 à 1000.

11°) Procédé selon la revendicaiton 10, caractérisé en ce que la phosphine utilisée est la même que celle présente dans le couple phosphine/palladium.

12°) Procédé selon l'une quelconque des revendications 1 à 11, caractérisé en ce que l'on conduit la réaction dans un solvant organique inerte.

13°) Procédé selon la revendication 12, caractérisé en ce que l'on utilise comme solvant organique la base azotée tertiaire utilisée comme accepteur d'hydracide.

14°) Procédé selon la revendication 11, caractérisé en ce que l'on utilise comme solvant organique un hydrocarbure aliphatique saturé, un hydrocarbure cycloaliphatique saturé, un hydrocarbure aromatique, un hydrocarbure aromatique halogéné, un éther, un lactame.

15°) Procédé selon la revendication 11, caractérisé en ce que l'on utilise comme solvant organique le composé halogénoaromatique dont dérive l'halogénoarènechrometricarbonyle.

Office européen des brevets

# RAPPORT DE RECHERCHE EUROPEENNE

Numero de la demande

EP 86 42 0158

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int. Cl 4) |
|---|---|---|---|
| D,A | EP-A-0 109 606 (BAYER)<br>* Revendications *<br>--- | 1 | C 07 C 45/49<br>C 07 C 47/52 |
| D,A | US-A-3 960 932 (R.F. HECK)<br>* Revendications *<br>----- | 1 | |

| DOMAINES TECHNIQUES RECHERCHES (Int. Cl.4) |
|---|
| C 07 C 45/00<br>C 07 C 47/00 |

Le present rapport de recherche a ete etabli pour toutes les revendications

| Lieu de la recherche | Date d'achevement de la recherche | Examinateur |
|---|---|---|
| LA HAYE | 19-08-1986 | BONNEVALLE E.I.H. |

CATEGORIE DES DOCUMENTS CITES

X : particulièrement pertinent à lui seul
Y : particulièrement pertinent en combinaison avec un autre document de la même categorie
A : arriere-plan technologique
O : divulgation non-ecrite
P : document intercalaire

T : théorie ou principe à la base de l'invention
E : document de brevet antérieur, mais publie à la date de dépôt ou après cette date
D : cité dans la demande
L : cité pour d'autres raisons

& : membre de la même famille, document correspondant

OEB Form 1503 03 82